# EUROPEAN PATENT SPECIFICATION

(11) **EP 1 648 532 B1**
(45) Date of publication and mention of the grant of the patent: **09.12.2009**
(21) Application number: 04742264.7
(22) Date of filing: 30.07.2004
(51) Int. Cl.: A61L 27/14, A61L 27/54, A61L 27/58

(54) **MULTIFUNCTIONAL IMPLANT DEVICE**
MULTIFUNKTIONALES IMPLANTAT
DISPOSITIF D'IMPLANT MULTIFONCTIONNEL

(30) Priority: 31.07.2003 FI 20031120
(43) Date of publication of application: 26.04.2006
(73) Proprietor: BIORETEC OY, 33720 Tampere (FI)
(72) Inventor: ASHAMMAKHI, Nureddin, Staffordshire ST4 6BY (GB); TÖRMÄLÄ, Pertti, FIN-33300 Tampere (FI)
(74) Representative: Hakola, Unto Tapani
(86) International application number: PCT/FI2004/050115
(87) International publication number: WO 2005/009496

(56) References cited:
- WO-A1-00/64516
- WO-A1-02/098307
- WO-A2-03/043576
- US-B1- 6 579 533

## Description

### FIELD OF THE INVENTION

The present invention relates generally to the treatment of disorders of skeletal tissue, to its regeneration and remodeling, and specifically, to devices and methods for inhibiting bone resorption and improving bone formation, in the form of fixation devices or supporting a prosthetic implant.

### BACKGROUND OF THE INVENTION

The basis of enhancing bone repair and regeneration is based principally on the use of 1) implant materials, 2) osteoinductive molecules, 3) osteoconductive particles or materials such as bone grafts, and ceramics such as HA, TCP or bioactive glass, etc. Since successful bone repair and regeneration involves various stages where biological and biomechanical factors interact to bring about the ultimate result of bone union, many factors in this complex interplay have to be addressed. Successful bone repair in some pathological conditions or in situations where bone healing may be delayed due to factors such as age, disease or drugs, are more challenging.

So far, replacing or supplementing fractured, damaged, or degenerated mammalian skeletal bone is based often on the use of biocompatible materials. Traditionally, these implants are of the permanent type that reside in the body without absorption. Besides associated risks of infection, loosening and osteopenia (due to stress protection), they are also poorly integrated into the bone. Factors that influence long-term implant viability include material type used, bone fixation method, implant location, surgical skill, patient age, weight and medical condition. A plethora of devices have been constructed attempting to optimize these variables involved in producing an increase in bone fusion.

Common materials that are used to manufacture prosthetic implant devices include ceramics, polymers and metals and their composites. Currently, metallic materials afford the highest mechanical properties necessary for use as skeletal prosthetic implants but they are much more rigid than the bone itself with the risk of stress shielding, osteopenia, weakening of the bone and risk of fracture or loosening. Frequently used metals include, titanium and titanium alloy, stainless steel, gold, cobalt-chromium alloys, tungsten, tantalum, as well as similar alloys.

Titanium is popular in the implant field because of its said superior corrosion resistance, biocompatibility, and physical and mechanical properties compared to other metals. However, recently some particles of titanium have been found in lymph nodes, and thus a search for a better material or radical change in the way that addresses the problem of treatment of bone needs to be developed. Also, a significant drawback to titanium implants is the tendency to loosen over time.

There are three typical prevailing methods for securing metal prosthetic devices in the human body: press-fitting the device in bone, cementing them to an adjoining bone with methacrylate-type adhesives, or affixing in place with screws. All methods require a high degree of surgical skill. For example, a press-fitted implant must be placed into surgically prepared bone so that optimal metal to bone surface area is achieved. Patient bone geometry significantly influences the success of press-fitted implants and can limit their usefulness as well as longevity. Similar problems occur with cemented implants. Furthermore, the cement itself is prone to stress fractures and is the ones commonly used are not bio-absorbable. Therefore, all methods are associated to varying degrees with cell lysis next to the implant surface with concomitant fibrotic tissue formation, prosthetic loosening, and ultimate failure of the device.

Bone itself is not a static but a dynamic tissue that undergoes turnover and remodeling. Bone is a living tissue whose cells interact with the biomechanical factors to adjust itself into a right remodeling line where either bone formation or bone resorption is the ultimate result in defined area at defined point in time. Even a well-suited implant at the time of surgery (using the principles of carpentry), such an implant can be found to be loosed after some time because of bone resorption. Thus, there is a clear need to address the implant-bone interface in a dynamic scale that involves both the implant and the tissue and timeframe.

Currently, methods are being developed to produce osteointegration of bone to metal in order to obviate the need for bone cements. Osteointegration is defined as bone growth directly adjacent to an implant without an intermediate fibrous tissue layer. This type of fixation avoids many complications associated with adhesives and theoretically would result in the strongest possible implant-to-bone bond. One common method is to roughen a metal surface creating a micro or macro-porous structure through which bone may attach or grow. Several implant device designs have been created attempting to produce a textured metal surface that will allow direct bone attachment. US Patents 5,609,635 and 5,658,333 are a couple of examples of these devices.

Metallic implant surfaces are also commonly coated with microporous ceramics such as hydroxyapatite (HA) or beta-tricalcium phosphate (beta-TCP), as disclosed for example in US Patents 4,960,646 and 4,846,837. The HA coatings increase the mean interface strength of titanium implants (see Cook et al., Clin. Ortho. Rel. Res., 232, p. 225, 1988), rapid bone growth and increased osteointegration (see Sakkers et. al., J. Biomed. Mater. Res., 26, p. 265, 1997). Optimal HA coating thickness ranges from 50-100 microns (see Thomas, Orthopedics, 17, p. 267-278, 1994). However, if the coating is too thick the interface it may become brittle. Despite the higher success rate of prosthetic devices coated with HA as compared to earlier implantation methods, failure over time still occurs. As said above, the problem is multifaceted and it necessitates an in-depth insight to develop a means that addresses many factors that come into play to affect the outcome of prosthetic devices, and among them the time-frame being an important factor. Although it is important that the surgeon should create an exact implant fit into bone allowing the metal and bone surfaces to have maximum contact at the time of surgery, things are dynamic and should the bone tissue not addressed in the proper biological and biomechanical language, it may turn the exactly fit implant into a loose one due to bone resorption. Also, fibrous tissue formation develops in some cases regardless of coating type used.

To enhance bone formation, one way is to use osteoinductive proteins (for example see Cole et. al., Clin. Ortho. Rel. Res., 345, p.21-228, 1997). Osteoinductive proteins are signaling molecules that stimulate new bone production. These proteins include PDGF, IGF-I, IGF-II, FGF, TGF-beta and associated family members. The most effective bone formation-inducing factors are the bone morphogenetic proteins (BMPs).

The BMPs represent a TGF-beta super-family subset. Over 15 different BMPs have been identified. Most members of this TGF-beta subfamily stimulate the cascade of events that lead to new bone formation (for example U.S. Pat. Nos. 5,652,118; and 5,714,589, reviewed in J. Bone Min. Res., 1993, v8, suppl-2, p. s565-s572). These processes include stimulating mesenchymal cell migration, osteoconductive matrix disposition, osteoprogenitor cell proliferation and differentiation into bone producing cells. Effort, therefore, has focused on BMP proteins because of their central role in bone growth and their known ability to produce bone growth next to implants e.g.titanium (see Cole et. al., Clin. Ortho. Rel. Res., 345, p. 219-228, 1997). One such method claims achievement of a strong bond between existing bone and the prosthesis by coating the prosthetic device with an osteogenic protein (see U.S. Pat. No. 5,344,654).

In U.S. Pat. No. 5,609,635, a method is described for the design of a spinal fusion device comprised of wire mesh infused with osteoinductive molecules. This device is intended solely for use in spinal fusions and is not designed for use with other prosthetic implants intended for use in other body areas. It is also not designed to be attached to orthopedic implants.

As in all forms of treatment, the goal is for selective beneficial effects on the target, in this case bone cells. For established osteoporosis, effective therapies will be those that stimulate osteoblast accumulation, proliferation and differentiation. There are a number of growth regulatory factors in bone that satisfactorily do this, including fibroblast growth factor-I (FGF-I), IGF-I and -II, BMPs, TGFβ and PDGF. Unfortunately, they also have potential toxic effects on other tissues. Effective delivery mechanism to enhance the concentrations of these growth regulatory factors locally in the bone microenvironment would be a desirable goal of any drug delivery mechanism (Gregory R. Mundy M.D. Pathogenesis of osteoporosis and challenges for drug delivery. Advanced Drug Delivery Reviews Volume 42, Issue 3, 31 August 2000, Pages 165-173).

One way to enhance bone tissue formation is the use of osteoconductive factors (see U.S. Pat. No. 5,707,962). One experienced in the art realizes that osteoconductive factors are those that create a favorable environment for new bone growth, most commonly by providing a scaffold for bone ingrowth. The clearest example of an osteoconductive factor is the extracellular matrix protein, collagen. Examples of other important osteoconductive materials are also the ceramics such as HA, TCP and bioactive glass.

Ways and factors mentioned so far, do, however, address only one facet of the complex process of ultimate remodeled bone formation. The other part should address the inhibition of osteolysis, a commonly-seen problem with prosthetic devices, with age, etc. As it is well-known in the literature that this process is conducted by the function of bone-resorbing cells known as osteoclasts. It is also well-known that these cells can be inhibited with agents known as bisphosphonates. Bisphosphonates are known to reduce the rate of bone turnover, reduce the rate at which new bone remodeling units are formed; reduce the depth of resorption; and produce a positive bone balance at individual remodeling units, resulting in an increase in bone mass over time (Watts NB, Treatment of osteoporosis with bisphosphonates. Endocrinol Metab Clin N Amer. 1998; 27: 419-439) . The use of bisphosphonate may thus also aid implant success (see U.S. Pat. No. 5,733,564).

It was indicated that there is a need for the development of medical management of periprosthetic osteolysis. Recently, it was demonstrated that bisphosphonates such as pamidronate induce specific apoptosis-related pathways in macrophages and indicated that this contributes data for a rational approach in the treatment and/or prevention of periprosthetic osteolysis (Huk OL, Zukor DJ, Antoniou J, Petit A. Effect of pamidronate on the stimulation of macrophage TNF-alpha release by ultra-high-molocular-weight polyethylene particles: a role for apoptosis. J Orthop Res. 2003 Jan;21(1):81-7.).

Anti-osteolytic bisphosphonates administered orally have the problem of poor GIT absorption and need for higher doses. GI upset the most common complaint seen with bisphosphonates. Although no significant side effects of alendronate compared to placebo emerged in the randomized clinical trials, postmarketing data indicated that esophagitis was a potentially serious side effect that occurs in a small percentage of patients. As of 1996, 475,000 patients had been treated with alendronate; 1,213 adverse reports had been received and, of these, 199 had adverse effects related to the esophagus. In 51 of these patients, the side-effects were rated as serious or severe (de Groen PC, Lubbe DF, Hirsch LJ, et al. Esophagitis associated with the use of alendronate. N Engl J Med. 1996; 335: 1016-1021). Specifically, chemical esophagitis was noted with erosions, ulceration or an inflammatory exudates (American Medical Association (http://www.ama-assn.org accessed on 15 Mar 2003)). It was thus advised that absorption of alendronate and risedronate is best if they are taken when arising in the morning, with 170-227 ml (6-8 oz) of water. The risk of esophagitis is said to be reduced if the patient remains upright for 30 minutes, and until the first food of the day has been ingested. Alendronate is contraindicated in patients with abnormalities of the esophagus that delay esophageal emptying, such as stricture or achalasia (de Groen PC, Lubbe DF, Hirsch LJ, et al. Esophagitis associated with the use of alendronate. N Engl J Med. 1996; 335: 1016-1021). These problems thus present a limitation on the use of bisphosphonates according to this mode of therapy and to a certain extent where local problem is addressed, there is a clear need to accomplish the drug delivery locally (to avoid GI problems).

It has been suggested to deliver bisphosphonates locally in the body. The use of bisphosphonates in implantable materials is known from the following publications:

International publication WO 00/64516 describes a method for controlled delivery of bisphosphonates for treatment of osteoporosis. The publication mentions a delivery device having the active agent in a bioresorbable matrix. The delivery device is implanted subcutaneously to allow sustained release of the bisphosphonate over an extended period of time. The device is intended as an alternative to oral administration, but it has no bone augmentation function. US patent 6214049 shows a fibrillar metal wire (e.g. of titanium) attached to a prosthetic device core and which can be coated with biodegradable polymer, which may contain various osteoconductive and osteoinductive factors. Osteoclast inhibitors such as bisphosphonate are mentioned as one example.

Local delivery of bisphosphonate by means of bone graft substitutes or extenders or autogenous or allogenic bone grafts is described in publications WO 02/062351 and WO 02/080933. These materials may also contain a carrier medium of a bioresorbable polymer, such as PGA and PLLA in the form of mouldable liquid, cement, putty, gel, flexible sheets, mesh or sponge. The bisphosphonate and the carrier alone may also be used for local delivery. The carrier may further contain other factors contributing to bone healing, such as growth factors.

International publication WO 03/030956 describes an improved demineralized bone matrix (DBM), which may contain biodegradable polymers acting mainly as diffusion barriers to degradative enzymes or retarding diffusion of the active factors from the implant site. The DBM composition may also be used as a drug delivery device and it may contain bisphosphonates for this purpose. The agent to be delivered is absorbed or otherwise associated with the DBM itself and the role of the polymer is to act as barrier.

The best combination that addresses many key-players in the process of ultimately successful bone formation, bone healing, regeneration and repair is thus needed: 1) The use of an implant that has strength properties and modulus close to that of bone may avoid the consequences associated with rigidity, stress protection and poor bone healing or even osteolysis; 2) The use of osteoinductive biomolecules to enhance and accelerate bone wound healing (fracture) especially in situations where this can be retarded such as in old age, systemic disease such as renal failure or drugs such as steroids or radiation treatment; 3) use of osteoconductive materials such as beta-TCP or bioactive glass; and 4) the use of anti-osteolytic agents such as bisphosphonates.

This will lead to the development of an implant that mimics the structure of bone itself. Bone tissue itself is a composite material made up of fibers of collagen running through hydroxyapatite, Ca₅(PO₄)₃OH. Hydroxyapatite constitutes about 70% of bone tissue. However HA itself is not resorbable and may lead to fibrous tissue formation.β-TCP & absorbable bioactive glass may be better alternatives.

Various matrices have been developed to contain and release bioactive peptides for osteo-induction, bone morphogenetic protein-molecules, as the matrix degrades. Organic polymers such as polylactides, polyglycolides, polyanhydrides, and polyorthoesters, which readily hydrolyze in the body into inert monomers, have been used as matrices (see U.S. Pat. Nos. 4,563,489; 5,629,009; and 4,526,909). The efficiency of BMP-release from polymer matrices depends on the matrices resorption rate, density, and pore size. Monomer type and their relative ratios in the matrix influence these characteristics. Polylactic and polyglycolic acid copolymers, BMP sequestering agents, and osteoinductive factors provide the necessary qualities for a BMP delivery system (see U.S. Pat. No. 5,597,897). Alginate, poly(ethylene glycol), polyoxyethylene oxide, carboxyvinyl polymer, and poly (vinyl alcohol) are additional polymer examples that optimize BMP-bone-growth-induction by temporally sequestering the growth factors (see U.S. Pat. No. 5,597,897). However, this approach adds only one factor in bone formation/bone resorption balancing process.

Non-synthetic matrix proteins like collagen, glycosaminoglycans, and hyaluronic acid, which are enzymatically digested in the body, have also been used to deliver BMPs to bone areas (for example U.S. Pat. Nos. 5,645,591; and 5,683,459). In human bone, collagen serves as the natural carrier for BMPs and in a way as an osteoconductive scaffold for bone formation. Demineralized bone in which the main components are collagen and BMPs has been used successfully as a bone graft material (see U.S. Pat. No. 5,236,456). The natural, or synthetic, polymer matrix systems described herein are moldable and release BMPs in the required fashion; however, used alone these polymers serve only as a scaffold for new bone formation. For example, U.S. Pat. Nos. 5,683,459 and 5,366,509 describe an apparatus, useful for bone graft substitute, composed of BMPs injected into a porous polylactide and hyaluronic acid meshwork. Furthermore, an osteogenic device capable of inducing endochondral bone formation when implanted in the mammalian body has been disclosed (see U.S. Pat. No. 5,645,591); this device is composed of an osteogenic protein dispersed within a porous collagen and glycosaminoglycan matrix. These types of devices were designed as an alternative bone graft material to replace the more invasive autograft procedures currently used. These devices by themselves would not work well probably due to their brittle nature. There is a need to develop a composite polymer product that provides proper elasticity, proper modulus and strength which is maintened for prolonged time than using a rapidly-degrading polymer.

Proper implant load distribution is yet another characteristic important for correct prosthetic function, such as described for example in U.S. patents Nos. 5,639,237, and 5,360,446. U.S. Pat. No. 5,458,653 describes a prosthetic device coated with a bioabsorbable polymer in specific implant regions to, theoretically, better distribute the load placed upon it. Many other endosseous dental implants with shapes attempting to distribute load including helical wires, tripods, screws and hollow baskets have also been used. The clinical success of all these implant types is dependent on placement site, implant fit and the extent of fibrous tissue formation around the implant preventing direct bone contact.

Hence there is a need to prevent as reduce fibrous tissue formation around implant. Upon biodegradation, fibrous tissue usually forms. The use of osteoconductive and/or osteoinductive agents thus is needed to enhance bone formation rather than fibrous tissue formation.

Despite the plethora of prior art approaches to securing an implanted structure into mammalian bone, or having successful bone formation and/or preventing osteolysis, there is a need in the surgical arts for improving the strength and integrity of the bone that surrounds and attaches to a prosthetic implant device and/or treat bone fractures, metastases or defects. Furthermore, there is a need in is art for a device that is multifunctional in terms of having bone treatment (fixation, repair or/and regeneration) function, osteoinductive function, osteoconductive function and anti-osteolytic function.

Up to date, no implants are known that would possess the above-mentioned advantageous properties.

### SUMMARY OF THE INVENTION

A method and apparatus for bone tissue management in a mammalian body is presented. A primary application of the present invention is to fix fractures and osteotomies, and support a prosthetic multifunctional implant device. The implant device comprises a biocompatible bioresorbable polymer as a matrix, a reinforcing biocompatible and bioresorbable, structure in close association with said matrix, and at least a bisphosphonate in said matrix. The mechanical strength of the implant device can be achieved by the use of self-reinforcement technique or other reinforcing technique.

As will be seen, the present invention provides a structure for achieving durable bone formation that has a better quality in terms of bone structure, mineral density and function (strength). In addition, the present invention provides a novel way to augment bone formation for a variety of applications in bone management.

To increase the multifunctionality, the implant can comprise: 1) a biocompatible bioresorbable polymer forming a matrix 2) osteoinductive and/or osteroinductive material in said matrix, and 3) a bisphosphonate in said matrix. In addition, one or more molecules that function as nutrients, blood-clotting factors, angiogenic factors, or trace elements, can be included in the matrix.

The matrix is in close association with a reinforcing structure made of biocompatible bioresorbable polymer, either of the same material or of different material in view of chemical composition. In some cases the matrix may be the reinforcing structure itself, where the increased strength compared with the raw material is created by means of manufacturing technique, for example orientation and fibrillation of the polymer material so that nearly the whole mass of material has been oriented in desired way (e.g. U.S. Patent No. 4,968,317). In this case the term matrix is understood as material capable of incorporating various substances, e.g. active agents such as antiostelytic agents and osteoinductive and/or osteoconductive material, and acting as carrier for them. If the reinforcing structure and the matrix exist as discrete areas in the implant device, the reinforcing structure possesses greater strength than the matrix, which may be due to different chemical composition, or different physical structure if the chemical composition is the same. The reinforcing portion can exist inside the matrix as discrete areas, such as elongate fibers, which may be different in composition or created by self-reinforcing technique form the same material as the matrix. Suitable self-reinforcing techniques creating areas of different physical properties starting from the same raw material within the implant device are good examples of the latter alternative.

The matrix can further exist as a coating in an implant device where the core is formed of biocompatible bioresorbable polymer material and acts as the reinforcing portion. The matrix can also be associated with the implant device as a separate piece, e.g. it may be wrapped around a resorbable implant device as a filament, mesh, sheet, or the like. In this case the piece need not necessarily have a reinforcing portion, because the implant device itself imparts strength to the combination, and the separate piece comprises matrix and the bisphosphonate.

Furthermore, the implant may comprise an osteoinductive-protein- sequestering agent comprising monomeric and polymeric units of hyaluronic acid, alginate, ethylene glycol, polyoxyethylene oxide, carboxyvinyl polymer, and vinyl alcohol. The agent can be also the polymers collagen or chitosan. The polymer may also be composed of a composite of synthetic or naturally occurring polymers comprising collagen and glycosaminoglycan. These materials act as carriers for the osteoinductive proteins and can be in the form of coating on the implant body or filled in pores, openings or channels in the body, which is an advantageous way of incorporating thermally sensitive proteins to the implant device.

Regarding the type of implant, resorbable polymers have been developed in a way that can be formed into osteofixation devices especially in bones where weight-bearing or mechanical demands are minimal. Hence, such resorbable devices have to have appreciable strength to rely on in mechanically-demanding fixation purposes. With the use of advanced manufacturing methods, such as self-reinforcing technology it is possible to manufacture such reliable devices. (Plast. Reconstr. Surg, review Ashammakhi; Pertti Törmälä, Clinical Materials 1992; U.S. Patent No. 4,743,257 to Törmälä et al.; U.S. Patent No. 4,968,317 to Törmälä et al. and U.S. Patent No. 6,503,278 to Pohjonen et al.).

It is also known that the inclusion of other agents in the structure of the resorbable polymeric device may lower the strength properties and renders it more elastic. Thus the use of advanced methods of self-reinforcement is a clear advantage in manufacturing successful multifunctional resorbable implant devices.

With the use of such resorbable devices that contain bisphosphonates, the problem of poor GIT absorption and need for higher doses can thus be avoided or at least reduced.

With the help of the implant device according to the invention, the efficiency of the above-described multifunctional therapy may also be increased and the supplement of the therapy to the area where it is needed to act at, such as around implants sites of metastasis, osteolysis or for fixation of bones can be achieved.

These and other features, aspects, and advantages of the present invention will be apparent from the accompanying drawings and from the detailed description and appended claims that follow.

### BRIEF DESCRIPTION OF THE DRAWINGS

Fig. 1 shows a first embodiment of the form of the implant device according to the invention,
Fig. 2 shows a second embodiment of the form,
Fig. 3 shows a third embodiment of the form,
Fig. 4 shows one embodiment of the relationship between the matrix and reinforcement,
Fig. 5 shows a second embodiment of the relationship,
Fig. 6 shows a third embodiment of the relationship,
Fig. 7 shows a fourth embodiment, and
Fig. 8 shows a fifth embodiment.

### DETAILED DESCRIPTION OF THE INVENTION

The implant device of the invention has at least the following components:
Matrix polymer

The matrix polymer is biocompatible and bioresorbale and acts as carrier for various agents and materials that contribute to the multifunctionality of the implant. Resorbable polymers that can be used are listed e.g. in table 1 of U.S.Patent No. 4,968,317, and those listed in table 1 of European patent 442911,.

The resorbabe polymers include the following:

| | |
|---|---|
| 1. | Polyglycolide (PGA) |
| | |
| | Copolymers of glycolide |
| 2. | Glycolide/lactide copolymers (PGA/PLA) |
| 3. | Glycolide/trimethylene carbonate copolymers (PGA/TMC) |
| | |
| | Polylactides (PLA) |
| | |
| | Stereoisomers and copolymers of PLA |
| 4. | Poly-L-lactide (PLLA) |
| 5. | Poly-D-lactide (PDLA) |
| 6. | Poly-DL-lactide (PDLLA) |
| 7. | L-lactide/DL-lactide copolymers |
| | L-lactide/D-lactide copolymers |
| | |
| | Copolymers of PLA |
| 8. | Lactide/tetramethylene glycolide copolymers |
| 9. | Lactide/trimethylene carbonate copolymers |
| 10. | Lactide/δ-valerolactone copolymers |
| 11. | Lactide/ε-caprolactone copolymers |
| 12. | Polydepsipeptides (glycine-DL-lactide copolymer) |
| 13. | PLA/ethylene oxide copolymers |
| | |
| 14. | Asymmetrically 3,6-substituted poly-1,4-dioxane-2,5-diones |
| | |
| 15. | Poly-β-hydroxybutyrate (PHBA) |
| 16. | PHBA/β-hydroxyvalerate copolymers (PHBA/PHVA) |
| | |
| 17. | Poly-β-hydroxypropionate (PHPA) |
| 18. | Poly-β-dioxanone (PDS) |
| 19. | Poly-δ-valerolactone |
| 20. | Poly-ε-caprolactone |
| 21. | Methylmethacrylate-N-vinylpyrrolidone copolymers |
| 22. | Polyesteramides |
| 23. | Polyesters of oxalic acid |
| 24. | Polydihydropyranes |
| 25. | Polyalkyl-2-cyanoacrylates |
| 26. | Polyurethanes (PU) |
| 27. | Polyvinyl alcohol (PVA) |
| 28. | Polypeptides |
| 29. | Poly-β-maleic acid (PMLA) |
| 30. | Poly-β-alcanoic acids |
| 31. | Polyethylene oxide (PEO) |
| 32. | Chitin polymers (derivatives of chitin) |

The above list is not meant to be exhaustive.

### Reinforcing structure

Matrix polymer is in close association with a reinforcing structure that contributes to the strength of the implant device, which is an important factor in bone fixation and other similar applications. A special case is the function of the matrix both as the carrier material and reinforcing structure, due to self-reinforcing technique during the manufacture of the implant device. Such techniques are based on mechanical modification of the polymeric raw material, and may include orientation and fibrillation of partly crystalline materials according to above-mentioned U.S. Patent 4,968,317, or mechanical modification of entirely amorphous materials by molecular orientation of the material, according to U.S. Patent 6,503,278,.

Another alternative is creating discrete areas of matrix and reinforcing structure in the implant device, and at least the matrix contains at the same time active agents and materials discussed above. The reinforcing structure may be of the same chemical composition as the matrix and be embedded in the same. An example of such a composite structure is disclosed e.g. in U.S.Patent No. 4,743,257; This structure is also termed "self-reinforced" because of the common origin of both matrix and the reinforcing structure.

The discrete areas of matrix and reinforcing structure can be composed of chemically different polymers, both being biocompatible and bioresorbable, and the polymer of the reinforcing structure being selected because of its mechanical properties (strength).

It is also possible that the reinforcing structure can be bioabsorbable inorganic materials, for example in the form of fibers of bioabsorbable bioactive glass, as described in U.S.Patent No. 6,406,498, In this case the bioactive glass may serve at the same time as osteoconductive material.

Further, the reinforcing structure can be the mass of the implant body having a coating which consists of matrix polymer. This matrix polymer acts as carrier for the above-mentioned active agents and materials. It is possible that the body of the implant device is of different bioresorbable material and is itself reinforced by some of the above-mentioned techniques. The matrix polymer of the coating can be in this case chitosan (a derivative of chitin) for example. The matrix polymer acting as carrier can be, alternatively to or additionally to being in the form of coating, filled in pores, channels or openings of the implant body.

Finally, the reinforcing structure may be an implant body on which the matrix polymer containing the above-mentioned active agents is fitted as a separate material piece, for example by winding, wrapping etc. The matrix may be in this case a filament, mesh, sheet, or the like, relatively flexible construction. Also in this case the structure of the implant body may be reinforced by any technique discussed above.

Bisphosphonate

Bisphosphonates are structural analogs of pyrophosphates. They have a pharmacologic activity specific for bone, due to the strong chemical affinity of bisphosphonates for hydroxyapatite, a major inorganic component of bone (see also Watts WB:Bisphosphonates therapy for postmenopausal osteoporosis. South Med J. 1992;85(Suppl):2-31.).

Bisphosphonates have the following general formula:

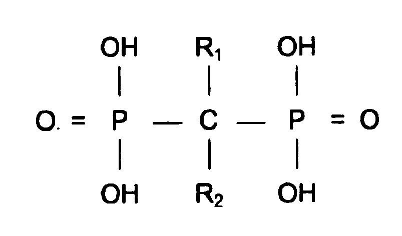

Substitution of different side chains for hydrogen at locations R₁ and R₂ changes the *in vitro* potency and side effect profile of the compound. Short alkyl or halide side chains (e.g., etidronate, clodronate) characterize first generation bisphosphonates. Second generation bisphosphonates include aminobisphosphonates with an amino-terminal group (e.g., alendronate and pamidronate). Tiludronate has a cyclic side chain, not an amino terminal group, but is generally classified as a second-generation compound based on its time of development and potency. Third generation bisphosphonates have cyclic side chains (e.g., risedronate, ibandronate, zoledronate). The antiresorptive properties of bisphosphonates increase approximately tenfold between drug generations. (Watts NB. Treatment of osteoporosis with bisphosphonates Endocrinol Metab Clin N Amer. 1998;27:419-439).

Other known bisphosphonates include incardronate (cimadronate), olpadronate, piridronate, minodronate, neridronate, EB-1053 and YH529. The term "bisphosphonate" includes acids, salts, esters, hydrates and other solvates.

Any bisphosphonate mentioned above can be used in the matrix polymer. It is also possible that two or more different types of bisphosphonates are used in the same implant device.

### Osteoconductive material

The osteoconductive material that is used in the implant device can be any factor known to create a favorable environment for new bone growth, most commonly by providing a scaffold for bone ingrowth. The osteoconductive factors that can be used is the extracellular matrix protein, collagen. Examples of other important osteoconductive factors are also the ceramics such as HA (hydroxyapatite), TCP (beta-tricalcium phosphate) bioactive glass, and bone graft (autogenic, allogenic or xenogenic bone graft) or its derivative. Two or more of the above-mentioned factors can be used in combination.

### Osteoinductive material

The osteoinductive material that is used in the implant device can be any osteoinductive protein that is known to stimulate new bone production. These proteins include PDGF, IGF-I, IGF-II, FGF, TGF-beta and associated family members. The most effective bone formation-inducing factors are the bone morphogenetic proteins (BMPs). Angiogenic factors such as VEGF, PDGF, FGF etc. can also be incorporated to enhance / maintain bone formation process where suitable.

Two or more of the above-mentioned factors can be used in combination.

### Implant device

The implant device can take any form known in surgery in connection with bone repair and healing (fixation, regeneration/generation, augmentation). It can be in the form of screw, nail, pin, bolt, plate, rod, mesh, scaffold or filament or some combination of the above structures, in general any stiff or tough structure having sufficient strength over the required period of time after being placed in contact with a bone. It can be shapeable to desired form by bending (for example a plate) to fit the site during the operation, or flexible but of sufficient tensile strength, such as a filament. Further, the device can have a closed surface or certain porosity or holes passing through.

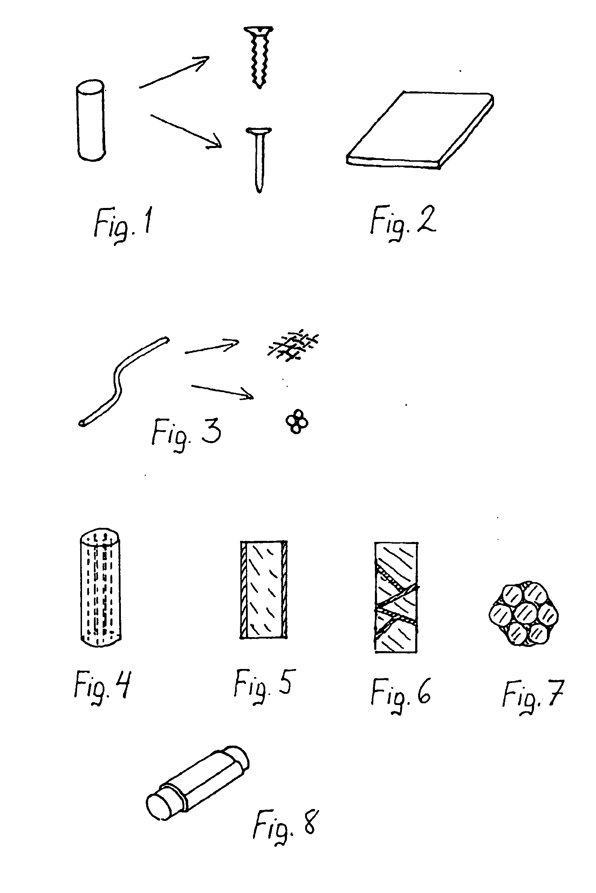

Fig. 1 shows a generally rod-shaped implant device, whose special shapes are screw and nail, which can be used as fixation devices for example. Fig. 2 shows a plate-shaped implant device. Fig. 3 shows a filament, of which a mesh (here in the form of woven fabric), or a thread or cord (shown in cross-section) can be formed. Fig. 4 shows a device where discrete reinforcing elements are embedded in the matrix. Fig. 5 shows a device in cross-section where a coating of matrix polymer exists on the implant body. Fig. 6 shows the alternative where channels inside an implant body are filled with matrix polymer. The same idea applies to implants where the body comprises pores or openings, which do not necessarily pass through the whole body. Fig. 7 shows the alternative where a matrix polymer is between filaments in a bundle (the polymer may also surround the bundle as a coating). Finally, Fig. 8 shows the alternative where a flexible structure comprising the matrix polymer is wrapped around an implant body.

In the embodiments of Figs. 5 to 7 the bisphosphonate can be in the matrix polymer in the coating, in the matrix polymer filling the channels, pores or openings, or in the matrix polymer between or around the filaments. The rest of the implant (the body or the filaments) is of another biocompatible bioresorbable material, preferably of another biocompatible bioresorbable polymer, which in turn may or may not contain reinforcing elements or areas, or may or may not be self-reinforced. The rest of implant may contain another active agent.

The bisphosphonate can also be in the rest of the implant, in which case this portion is of biocompatible bioresorbable polymer, which in turn contains reinforcing elements or areas, or is self-reinforced. The coating (Fig. 5) or the filling material (Fig. 6 or 7) may contain another active agent.

The implant device in one embodiment may have a composite of mesh and stem, where the stem can have the bisphosphonate in a matrix. An example of such a composite is a joint prosthesis, which is disclosed in U.S. Patent No. 6,113,640, where the fixation parts serving to fix the prosthesis to the bone could have the bisphosphonate.

Its should also be understood that the osteoconductive and/or osteoinductive material need not necessarily be in the same matrix as the bisphosphonate but they can be in another matrix phase but in the same implant device.

## Claims

1. A multifunctional implant device for bone augmentation function comprising at least more than one component in its structure, **characterized in that** it comprises
- a biocompatible bioresorbable polymer as a matrix;
- bisphosphonate capable of inhibiting osteoclasts in said matrix, and
- a reinforcing biocompatible bioresorbable structure in close association with the matrix in one of the following ways:
1) the biocompatible bioresorbable polymer of the implant device is self-reinforced;
2) the implant device comprises discrete reinforcing elements or areas in the matrix;
3) the matrix exists as coating in the implant device;
4) the matrix is filled in pores, channels or openings of the implant body;
5) the matrix is between filaments in a bundle;
6) the matrix is fitted as separate material piece on the implant body.

2. The implant device according to claim 1, **characterized in that** the matrix is self-reinforced by reinforcing elements or areas of the same bioresorbable polymer.

3. The implant device according to claim 1, **characterized in that** the matrix is reinforced by reinforcing elements or areas of different material, such as different bioresorbable polymer.

4. The implant device according to any of claims 1 to 3, **characterized in that** the implant device comprises also osteoconductive and/or osteoinductive material.

5. The implant device according to claim 4, **characterized in that** the osteoinductive material is one or several from the following: PDGF, IGF-I, IGF-II, FGF, TGF-beta, BMP, angiogenic factors.

6. The implant device according to claim 4 or 5, **characterized in that** the osteoconductive material is one or several from the following: collagen, HA, TCP, bioactive glass, bone graft or its derivative.

7. The implant device according to any of claims 1 to 6, **characterized in that** the implant device is a screw, nail, pin, bolt, plate, rod, mesh, filament, bundle of filaments, cord, or thread.

## Patentansprüche

1. Multifunktionsimplantatvorrichtung für die Knochenaugmentationsfunktion, wobei die Vorrichtung zumindest mehr als eine Komponente in ihrer Struktur umfasst, **dadurch gekennzeichnet, dass** sie Folgendes umfasst:
- ein biokompatibles, bioresorbierbares Polymer als eine Matrix;
- Bisphosphonat, das Osteoklasten in der Matrix unterbinden kann, und
- eine biokompatible, bioresorbierbare Verstärkungsstruktur in enger Verbindung mit der Matrix in einer der folgenden Weisen:
1) das biokompatible, bioresorbierbare Polymer der Implantatvorrichtung ist selbstverstärkt;
2) die Implantatvorrichtung umfasst separate Verstärkungselemente oder -bereiche in der Matrix;
3) die Matrix besteht als Überzug in der Implantatvorrichtung,
4) die Matrix ist in Poren, Kanäle oder Öffnungen des Implantatkörpers gefüllt;
5) die Matrix ist zwischen Filamenten in einem Bündel;
6) die Matrix ist als separates Materialstück auf dem Implantatkörper angebracht.

2. Implantatvorrichtung nach Anspruch 1, **dadurch gekennzeichnet, dass** die Matrix durch Verstärkungselemente oder -bereiche aus ein und demselben bioresorbierbaren Polymer selbstverstärkt ist.

3. Implantatvorrichtung nach Anspruch 1, **dadurch gekennzeichnet, dass** die Matrix durch Verstärkungselemente oder -bereiche aus unterschiedlichem Material, wie unterschiedlichen bioresorbierbaren Polymeren, verstärkt ist.

4. Implantatvorrichtung nach einem der Ansprüche 1 bis 3, **dadurch gekennzeichnet, dass** die Implantatvorrichtung außerdem osteokonduktives und/oder osteoinduktives Material umfasst.

5. Implantatvorrichtung nach Anspruch 4, **dadurch gekennzeichnet, dass** das osteoinduktive Material ein oder mehrere Materialien der folgenden ist: PDGF, IGF-I, IGF-II, FGF, TGF-beta, BMP, angiogenetische Faktoren.

6. Implantatvorrichtung nach Anspruch 4 oder 5, **dadurch gekennzeichnet, dass** das osteokonduktive Material ein oder mehrere Materialien der folgenden ist: Kollagen, HA, TCP, bioaktives Glas, Knochentransplantat oder dessen Derivat.

7. Implantatvorrichtung nach einem der Ansprüche 1 bis 6, **dadurch gekennzeichnet, dass** die Implantatvorrichtung eine Schraube, ein Nagel, ein Stift, ein Bolzen, eine Platte, eine Stange, ein Netz, ein Filament, ein Filamentbündel, eine Kordel oder ein Faden ist.

## Revendications

1. Dispositif d'implant multifonctionnel pour la fonction d'augmentation osseuse comprenant au moins plus d'un composant dans sa structure, **caractérisé en ce qu'**il comprend
- un polymère biorésorbable biocompatible comme matrice ;
- du diphosphonate capable d'inhiber les ostéoclastes dans ladite matrice, et
- une structure biorésorbable biocompatible de renfort en étroite association avec la matrice dans l'une des voies suivantes :
1) le polymère biorésorbable biocompatible du dispositif d'implant est auto-renforcé ;
2) le dispositif d'implant comprend des éléments ou des zones de renfort discrets/discrètes dans la matrice ;
3) la matrice existe comme revêtement dans le dispositif d'implant ;
4) la matrice est chargée dans des pores, des canaux ou des ouvertures du corps de l'implant ;
5) la matrice est située entre des filaments au sein d'un faisceau ;
6) la matrice est fixée comme pièce de matériau séparée sur le corps de l'implant.

2. Dispositif d'implant selon la revendication 1, **caractérisé en ce que** la matrice est auto-renforcée par des éléments ou des zones de renfort du même polymère biorésorbable.

3. Dispositif d'implant selon la revendication 1, **caractérisé en ce que** la matrice est renforcée par des éléments ou des zones de renfort d'un matériau différent, tel qu'un polymère biorésorbable différent.

4. Dispositif d'implant selon l'une quelconque des revendications 1 à 3, **caractérisé en ce que** le dispositif d'implant comprend également un matériau ostéoconducteur et/ou ostéo-inducteur.

5. Dispositif d'implant selon la revendication 4, **caractérisé en ce que** le matériau ostéo-inducteur est l'un ou plusieurs parmi les suivants : PDGF, IGF-I, IGF-II, FGF, TGF-bêta, BMP, et les facteurs angiogéniques.

6. Dispositif d'implant selon la revendication 4 ou 5, **caractérisé en ce que** le matériau ostéoconducteur est l'un ou plusieurs parmi ceux qui suivent : collagène, HA, TCP, verre bioactif, greffe osseuse ou son dérivé.

7. Dispositif d'implant selon l'une quelconque des revendications 1 à 6, **caractérisé en ce que** le dispositif d'implant est une vis, un clou, une aiguille, un boulon, une plaque, une barre, une maille, un filament, un faisceau de filaments, une corde, ou un fil.
